(19) Europäisches Patentamt
European Patent Office
Office européen des brevets

(11) **EP 2 170 302 B1**

(12) **EUROPEAN PATENT SPECIFICATION**

(45) Date of publication and mention
of the grant of the patent:
**11.04.2012 Bulletin 2012/15**

(21) Application number: **08719112.8**

(22) Date of filing: **07.03.2008**

(51) Int Cl.:
*A61K 9/51* (2006.01)     *A61K 47/48* (2006.01)

(86) International application number:
**PCT/HU2008/000028**

(87) International publication number:
**WO 2008/107729 (12.09.2008 Gazette 2008/37)**

(54) **SUSTAINED RELEASE BIONANOCOMPOSITES, A PROCESS FOR PRODUCING THE SAME AND USE THEREOF**

BIONANO-KOMPOSITE MIT VERZÖGERTER FREISETZUNG, VERFAHREN ZU IHRER HERSTELLUNG UND IHRE VERWENDUNG

BIONANOCOMPOSITES A LIBERATION PROLONGEE, LEUR PROCEDE DE FABRICATION ET LEUR UTILISATION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **08.03.2007 HU 0700203**

(43) Date of publication of application:
**07.04.2010 Bulletin 2010/14**

(73) Proprietors:
• **Szegedi Tudomanyegyetem**
**6720 Szeged (HU)**
• **Pannonpharma Gyógyszergyártó Kft.**
**7720 Pécsvárad (HU)**

(72) Inventors:
• **DÉKÁNY, Imre**
**H-6722 Szeged (HU)**
• **SZEKERES, Márta**
**H-6720 Szeged (HU)**

• **PALLAI, Zsolt**
**H-1221 Budapest (HU)**
• **BALI, László**
**H-1033 Budapest (HU)**

(74) Representative: **Kiss, Ildiko et al**
**DANUBIA Patent and Law Office LLC**
**POB 198**
**1368 Budapest (HU)**

(56) References cited:
**WO-A-2006/106520     US-A1- 2005 239 167**

• **BALABUSHEVITCH N G ET AL: "Encapsulation of proteins by layer-by-layer adsorption of polyelectrolytes onto protein aggregates: factors regulating the protein release." BIOTECHNOLOGY AND BIOENGINEERING NOV 2001, vol. 76, no. 3, November 2001 (2001-11), pages 207-213, XP002487925 ISSN: 0006-3592**

**Description**

Technical field of the invention

The present invention relates to a sustained release, core-shell structure, cytokine-containing bionanocomposite, a process for preparation thereof and use of said core-shell bionanocomposite in the preparation of a sustained release pharmaceutical composition..

Background of the invention

The formulation of biologically active proteins (e.g., enzymes, hormones, cytokines) as drugs poses a number of specific problems related to the preservation of biological activity through conservation of higher order protein structures. Secondary, tertiary and quaternary structure of the proteins are prerequisites of biological function, in addition to their primary structure. The decrease in biological activity due to conformational changes is more probable for proteins isolated from natural sources, than for recombinant proteins. Thus, sufficiently mild conditions are needed to ensure the stability of not only the primary but the higher structure against irreversible conformational changes.

Appropriate dosing and correct targeting are equally important in effective curing, in addition to the use of the possibly most effective drugs. The role of controlled release systems is to enhance drug efficiency and to distribute the drugs in time and space in the body according to the actual demand. The design of sustained drug release systems is one of the most current fields of drug formulation. Sustained release property of formulations is very important in two main aspects. On the hand, it allows to limit the number of dosing, which improves life quality of patients using injection or suppository formulations. At the same time it helps sustaining a relatively stable drug level in the organism, improving thereby the medical effect.

There are basically two possible ways to achieve sustained release. According to the first one, the dissolution of the drug is retarded by means of some physical processes. For example, dissolution of a drug from crystalline formulations is retarded by the large particle size of the crystals. Thus, grinding affects release kinetics. Another way is to apply chemical and colloid methods to achieve sustained release. For example, in case of tablet formulations polymer or other multilayered coatings ensure sustained therapic effect due to slow diffusion of the drug through the pores of the coatings, or due to pH- and/or temperature-dependent swelling of the coating polymer. Parenteral preparations contain drug particles of various size depending on the locus of administration. The appropriate drug particle size in case of suppositories or transdermal preparations depends mainly on the mechanism of absorption. The drug in these kinds of preparations is in the form of nano or microparticles, which protects the drug from premature decomposition and instant dissolution. The mechanisms of release vary from simple hindered diffusion through adsorption-equilibrium controlled dissolution to controlled disintegration of nano and microparticles or drug release via biodegradation of the matrix of the formulation. Research in pharmaceutical industry today is oriented to explore even more complex and innovative methods to satisfy increasing demands on specific release mechanisms. Sustained effect can last from a few hours to several days, but there are also commercially available drug formulations that are able to sustain the required drug level in an organism during months with no significant change.

Sustained release offers additional benefits as well. It protects the organism from the instant appearance of the drug in much too large concentrations (burst). Instant, large amounts of drugs may be deleterious, but at least they are unnecessary and clear out from the organism without any appreciable use. Sustanied release this way is an economic question in addition to medical effects.

There are various mechanisms known to prepare sustained release protein formulations.

The most widespread method is the so-called encapsulation method, i.e., coating with polymers/polyelectrolytes. The structure of the prepared capsules can be either of a core-shell type with multilayered polymer layers around the protein microparticle, or homogeneous sphere type with the protein evenly distributed in the pores of the polymer sphere. The immobilized protein agent is released gradually from these structures: either dissolves from the complex or liberates due to decomposition of the polymer matrix. There are four prevalent microcapsulation methods, as follows.

a) Phase-sepharation method is coacervation by using organic solvents. In this method a water-in-oil emulsion is prepared wherein the protein agent is dissolved in the aqueous phase. The emulsion droplets are stabilized by polymeric emulsifiers followed by the extraction of the oily phase by using another, high-tension organic solvent (freon, heptane, hexane or cyclohexane). Extraction of the total organic content from such preparations requires considerable energy and work input, which is the main drawback of the method (EP 0 052 510, SE 462 780, US 5,000,886 patents).

b) Spray-drying, spray-coating. The mixture of protein and polymer solutions is subjected to spray-drying resulting in the formation of composite granules. The proteins liberate from the uncoated microparticles relatively quickly, since the particles formed by this method have considerable porosity. Used PLGA (polymer of lactic acid and glycolic acid) for spray-coating to form core-shell particles improves release properties. Spray-drying at low temperature is appropriate to formulation of temperature sensitive proteins (US 5,019,400, WO 90/13780, US 4,166,800, US 4,568,559). The energy demand of the method is large and the particle size and particle size distribution is not controlled well.

c) Solvent evaporation method. An oil-in-water emulsion is prepared with the organic dispersed phase consisting of mixture of a polymer and the protein drug in an organic solvent. The polymer solidifies during evaporation of the organic solvent (during several hours) and the protein agent gets enclosed in the porous gel particle (US 5,407,609, US 4,384,975, WO 96/07399, US 4,652,441, US 5,407,609, US 5,654,010, WO 96/40074, WO 90/13780, US 5,589,167, US 6,861,064). The method has several drawbacks: large energy demand, organic solvents do not meet toxicological criteria, inapplicability for water soluble proteins, emulgeation process can cause protein denaturation due to high shear forces, emulsion stabilization methods are not effective enough to prevent particle aggregation.

d) Formation of gel-particles. The Medusa® process of Flamel Technologies Inc., is a formulation method of proteins, among them IFN-α, within nanospheres prepared by spontaneous association of two amino acids, leucine (hydrophobic) and glutamic acid (anionic). The size of the nanospheres is 20-50 nm, the water content is 95 %. The inner core (polyleucine chains) solubilizes the protein and the outer surface of the particles contains the glutamic acid side chains ensuring solvation of the nanoparticles in the aqueous environment. The drawback of the method is, that the protein agent (e.g. interferon) is added to the system in a separate infiltration step. The procedure is protected by more than 50 patents, among them US 5,904,936.

Nano- and microparticles that preserve the physico-chemical properties of the incorporated proteins can be prepared in an economic way from supercritical fluids, so that the bioavailability and the activity of the proteins remains maximal as well [N. Jovanovic, A. Bouchard, G. W. Hofland, G.-J. Witkamp, D. J. A. Crommelin, W. Jiskoot, Stabilization of Proteins in Dry Powder Formulations Using Supercritical Fluid Technology, Review, Pharmaceutical Research, 21, 1955-1969 (2004)]. However, protection of the formed primary particles is not solved. For sustained release use encapsulation procedures must be applied, mostly with PLGA matrices. Protein nanoparticles of 100 - 500 nm diameter can be prepared by using the SAS-EM (supercritical anti-solvent with enhanced mass transfer) technology. The drawback of the method is that the proteins are dissolved in non-aqueous solvents (i.e., lysozyme in dimethyl sulfoxide) and in addition, the supercritical method demands large energy and apparatus expenses. The SAS apparatus operates at high pressure. In SAS-EM, in addition, the injected protein solution is dispersed to form nanodroplets by the use of a vibrating surface where vibration is generated at ultrasound frequency [P. Chattopadhyay, R. B. Gupta, Protein Nanoparticles Formation by Supercritical Antisolvent with Enhanced Mass Transfer, AIChE Journal, 48, 235-244 (2002)].

Complex coacervation procedure was used to prepare spherical composite nanoparticles exploiting charge compensation mechanism between the protein agents (albumin, casein) and the polyelectrolytes (gelatine). Desolvation of proteins by simple coacervation methods (temperature, pH, solvent, or ionic strength generated coacervation) is used to prepare drug carrier protein nanoparticles. Survay of the procedures is found in C.S.S.R. Kumar (ed.) Biological and Pharmaceutical Nanomaterials (Nanotechnologies for life sciences, Volume 2, Wiley-VCH, Verlag GmbH & Co. KGaA, Weinheim, 2006), chapter 6. Peptide nanoparticles, p. 145. Coacervate particles are stabilized by chemical bonding using carbodiimide or glutaraldehyde crosslinkers. The drawback of the method is that the proteolytic degradation of the protein nanoparticles in vivo does not allow for long term (one week) sustained release applications.

Description of the preparation method of several sustained release Zn-protein aggregates can be found in literature [U. Gietza, T. Arvinte, M. Haner, U. Aebi, H. P. Merkle, Formulation of sustained release aqueous Zn-hirudin suspensions, European Journal of Pharmaceutical Sciences 11, 33-41 (2000)]. The proteins were precipitated using $ZnCl_2$ or $MgCl_2$ solutions at neutral pH (7 - 7.5). The structure of the microparticles of about 200 $\mu$m of diameter and the effectivity of precipitation as a function of solution pH and ionic strength were studied without an effort to explain the mechanisms behind the procedure. The precipitated protein microparticles were not coated by any protective layer. There are no data for drug release kinetics in the publication.

In general, object of the preparation of the core-shell micro- or nanoparticles has been the preparation of capsules into which drug molecules can be filled in a separate infiltration step. The basis of the preparation method is the layer-by-layer (L-b-L) or electrostatic self-assembly (ESA) method worked out originally for the case of preparation of multilayered nanostructures on flat macroscopic surfaces.

H. Möhwald and co-workers prepared micrometer-sized (10 $\mu$m diameter) enzyme crystals and adsorbed anionic (PSS, polystyrene sulfonate) and cationic (PAH, polyallylamine hydrochloride) polyelectrolytes alternately on the surface of the particles [N. G. Balabushevitch, G. B. Sukhorukov, N. A. Moroz, D. V. Volodkin, N. I. Larionova, E. Donath, H. Mohwald, Encapsulation of Proteins by Layer-by-Layer Adsorption of Polyelectrolytes onto Protein Aggregates: Factors Regulating the Protein Release, Biotechnology and Bioengeneering, 76, 207-213 (2001) and F. Caruso, D. Trau, H. Mohwald, R. Renneberg, Enzyme Encapsulation in Layer-by-Layer Engineered Polymer Multilayer Capsules, Langmuir, 16, 1485-1488 (2000)]. The enzyme was precipitated from 1 M K-acetate solution (pH=5) by cooling to 4 °C. The crystallized chymotrypsin retained biological activity, measured after liberating of the enzyme at pH=11 form the particles. Möhwald and co-workers had proved that multilayered polyelectrolyte structures can be formed on microparticle surfaces, besides flat macroscopic surfaces. The polyelectrolyte layers are permeable and various drug molecules can be infiltrated into the capsules via simple diffusion process. The synthesis of the composite core-shell particles requires stringent conditions (4 °C for precipitation, 2-3 washing steps per layer, 7-12 polyelectrolyte layers on average), which are economically less attractive. Release of the crystalline biomolecule from the core was measured during 24 hours and reached 30-70 %, depending on the number of layers and pH. At physiological pH (pH ~ 7) the release did not exceed 50 %.

Polyelectrolyte microcapsules using silicon dioxide particles as templates were prepared by Möhwlad and co-workers, which showed pH-dependent reversible swelling properties [T. Mauser, C. De jugnat, H. Möhwald, G. B. Sukhorukov, Microcapsules Made of Weak Polyelectrolytes: Templating and Stimuli-Responsive Properties, Langmuir, 22, 5888-5893].

Porous $CaCO_3$ particles were used to adsorb a protein agent onto the outer surface by Volodkin and co-workers [D. V. Volodkin, N. I. Larionova, G. B. Sukhorukov, Protein Encapsulation via Porous CaCO3 Microparticles Templating, Biomacromolecules, 5, 1962-1972 (2004)]. Electrostatic adsorption of polyelectrolytes was used to coat the composite particles and the inorganic template was extracted by the help of a chelate-forming agent resulting in core-shell structured microparticles.

H. Ai and co-workers prepared melamine formaldehyde microparticles to obtain polyelectrolyte microcapsules into which drug agents were infiltrated [H. Ai, J. J. Pink, X. Shuai, D. A. Boothman, J. Gao, Interactions between self assembled polyelectrolyte shells and tumor cells, © 2005 Wiley Periodicals, Inc., www.interscience.wiley.com, DOI: 10.1002/jbrm.a.30289]. By varying the type of the polyelectrolytes they investigated the interaction between cells and the outer surface of the shell.

The above mentioned methods of nanocomposite and microcomposite preparation contain the step of the dissolution of the template, which carry in itself the danger of protein denaturation or, if the protein is not included during the formation, it must be loaded into the particles posterior, in a separate procedure. The latter, infiltration procedure limits the protein loading efficiency. The size of the core-shell structures formed on the surface of NaCl-precipitated proteins [Möhwald and co-workers, supra] is in the micrometer range, because the layer structure preparation could be conducted on sufficiently stable aggregates only. Micrometer sized particle suspensions, however, can not be administered intravenously, thus, their use is restricted.

L. Jorgensen and co-workers analyze the methods of protein formulations (microspheres, nanoparticles, liposomes, emulsions, hydrogel based macroparticles) [L. Jorgensen, E. H. Moeller, M. van de Weert, H. M. Nielsen, S. Frokjaer, Preparing and evaluating delivery systems for proteins, European Journal of Pharmaceutical Sciences, doi: 10.1016/j.ejps.2006.05.00811] and state that many of important questions such as formulation stability, complete drug release, shelf stability, are not yet resolved.

A method for protein encapsulation is disclosed by Balabushevitch N.G. et al., where protein aggregates are covered with polyelectrolyte layers. Particularly, α-Chymotrypsin is encapsulated with two polyelectrolyte layers of poly (styrene sulfonate) or poly(allylamine hydrochloride) respectively [Balabushevitch N.G. et al., "Encapsulation of proteins by layer-by-layer adsorption of polyelectrolytes onto protein aggregates: factors regulating the protein release". Biotechnology and Bioengineering, 76, 207-213 (2001)].

WO2006/106520 discloses a method for encapsulation of active compounds like cytokines by complexation with an amphiphilic polymer like chitosan or alginate.

US2005/239167 discloses the fusion of interferon with albumin in order to increase the plasma stability of interferon.

Object of the invention

Our aim was to formulate biologically active proteins, mainly cytokines, in non-aggregated primary particles of 10 - 1000 nm diameter of relatively monodisperse distribution from which the proteins are released in their biologically active form according to linear kinetics in a prolonged interval of several days to one week.

Our invention is based on the recognition of the fact, that proteins precipitated from aqueous solution by using simple electrolytes can form nanoparticles controllably under specific conditions, and after stabilizing the said nanoparticles by using polyelectrolytes the resulting nanocomposites meet the following requirements:

    a) controlled particle size in the nano-range (1 to 1000 nm)
    b) stability against aggregation
    c) linear release kinetics during at least 3 days
    d) reversible protein precipitation in order to retain biological activity.

## Summary of the invention

The present invention is directed to a nanocomposite providing sustained release of bioactive proteins which comprises

a) a charge-carrying, bioactive protein core, precipitated reversibly in an ionic mechanism and
b) a polyelectrolyte shell around the said core consisting of two oppositely charged polyelectrolytes, forming 3 to 5 layers, where one of the polyelectrolytes has pH-dependent charges while the other polyelectrolyte has permanent, pH-independent charge.

Further, the present invention is directed to a procedure for the preparation of said nanocomposite wherein

i) a biocative protein or a protein mixture containing at least one bioactive protein

a) is dissolved in an aqueous medium at a pH below the isoelectric point of the bioactive protein, the pH being adjusted with appropriate buffer solution, and in the following a two- or three-valence anion containing electrolyte is added to the solution while stirring or
b) is dissolved in an aqueous medium at a pH above the isoelectric point of the bioactive protein, the pH being adjusted with appropriate buffer solution, and in the following a multivalent non-hydrolizing cation containing electrolyte is added to the solution while stirring,

ii) the precipitated protein of positive charge (according to point a) or of negative charge (according to point b) is separated by centrifugation,
iii) a solution of a first polyelectrolyte that is oppositely charged relative to the protein is added to the above protein sediment while mixing at a pH adjusted by a buffer to the pH of the precipitated protein,
iv) the polyelectrolyte-coated nanocomposite particles charged oppositely relative to the precipitated protein core are separated by centrifugation and optionally washed with buffer solution,
v) a solution of a second polyelectrolyte, charged oppositely relative to the first polyelectrolyte is added to the sediment at the same pH adjusted by buffer,
vi) the two-polyelectrolyte-coated nanocomposite particles having similar charge as the protein core are separated by centrifugation and optionally washed with buffer, and
vii) steps iii) - vi) are repeated until the desired number of layers is formed and, finally,
viii) sediment of the core-shell nanocomposite containing 3 to 5 coating layers as desired on the primary protein nanoparticle is freeze-dried.

In a further aspect the present invention is directed to a sustained release drug formulation which comprises a nanocomposite according to the invention and optionally other bioactive agent(s) and pharmaceutically acceptable inert carrier(s) and/or other excipient(s).
In a further aspect the present invention is directed to the use of said nanocomposite in the preparation of a sustained release drug formulation.

## Brief description of the drawings

Figure 1 shows schematically the core-shell nanocomposite structures prepared by the L-b-L method using anionic polyelectrolyte (polystyrene sulfonate, PSS (-)) and cationic polyelectrolyte (chitosan (+)) alternate coating layers on cytokine containing human serum albumin (cHSA (+)) core nanoparticles in 3 layers (A) and in 4 layers (B).
Figure 2 shows the changes in the electrokinetic potential (zeta potential, mV) as a function of the number of polyelectrolyte layers during the process of L-b-L coating of cytokine containing human serum albumin nanoparticles (cHSA) with the said polyelectrolytes. PSS means polystyrene sulfonate, Chit means chitosan.
Figure 3 shows a representative TEM picture of the cHSA-PSS/chitosan/PSS nanocomposite preparations. Panels A and B are pictures of the same preparation at different magnifications and focal distances.
Figure 4 shows the UV-VIS spectra of solutions of interferon (LE-EFN), PSS and (LE-IFN + PSS). Wavelength, $\lambda$ (nm) is plotted on horizontal, absorption (Abs) on vertical axes.
Figure 5 shows release kinetics curves of four-layered cHSA nanocomposites:

$\Delta$ - $\Delta$ is the cummulative release curve of the preparation Example 2 and ● - ● is that of Example 8 (from two independent experiments).

Figure 6 shows release kinetics curves of three-layered cHSA nanocomposites:

● - ● is the cummulative release curve of the preparation Example 1, ▲ - ▲ is that of Example 5 and o - o is that of Example 6.

Detailed description of the invention

The present invention thus provides a nanocomposite ensuring sustained release of bioactive proteins, which consists of

a) an ionically reversibly precipitated, charged primary protein nanoparticle core containing at least one bioactive protein and

b) 3 to 5 polyelectrolyte layers around said nanoparticle core prepared by alternate adsorption of two oppositely charged polyelectrolytes, the dissociation of one of which is pH-dependent, while the other polyelectrolyte is fully dissociated independently of pH.

The protein core preferably contains one or more cytokines as bioactive protein. As the chemical composition concerns, cytokines are glycoproteins that are produced in immune cells (leukocytes, macrophages, monocytes, etc.) in response to some stimuli such as virus infection. The primary core may contain cytokines originating preferably from leukocyte and even more preferably from human blood leukocyte, for example interferons (IFN), interleukins (IL), macrophage inflammation proteins (MIP), tumor necrosis factors (TNF), or may consist thereof. The protein core may contain biologically inactive carrier (vector) proteins as well, besides the bioactive proteins. The dosage of the nanocomposite can be adjusted by varying the ratio of bioactive to inactive proteins of the core.

According to the present invention the core forming protein is precipitated by an electrolyte, at least one of the ions of which is two- or three-valent. More specifically, it is precipitated by an electrolyte which contains two- or three-valent anion at a pH below the isoelectric point (iep) of the protein, or two- or three-valent non-hydrolyzing cation at a pH above the iep.

As an anion, whichever of $SO_4^{2-}$, $HPO_4^{2-}$, $CO_3^{2-}$, $PO_4^{3-}$ may be appropriate. As active precipitating cation, only $La^{3+}$ can be used. Other non-toxic higher valent cations undergo hydrolysis at higher pH values and complex oxide-hydroxide aggregate ions are formed onto which the proteins are flocculated and this way controlled size protein nanoparticles can not form. Non-hydrolyzing higher valence cations are in general heavy metal ions which are not suited for drug formulation purposes. During anionic precipitation positively charged protein nanoparticle cores, while during cationic precipitation negatively charged protein nanoparticles are formed.

According to the present invention the oppositely charged polyelectrolytes are situated alternately around the protein core, forming the so called "core-shell" nanostructure, which is formed by adsorption mechanism, according to the self assembly principle. Each polyelectrolyte layer is bound to the core or to the preceding polyelectrolyte layer via electrostatic interaction forces and reverses the surface charge. Thus, depending on the number of polyelectrolyte layers we can prepare bionanocomposites of positive or negative surface charge.

Any cationic-anionic polyelectrolyte pairs are appropriate to form the polyelectrolyte layers if one of the polyelectrolyte is a strong electrolyte (fully dissociated independent of the pH of the medium) and the other is weak (degree of dissociation depends on pH), and in addition, the pharmaceutical use of which is permitted. Ion-complexes formed by two strong polyelectrolytes are not permeable for macromolecules, because of the high charge density of the polyelectrolytes, unless the charges are screened by electrolytes. In the latter case, change in the ionic strength will alter the charge density. Two weak polyelectrolytes on the other hand do not form oppositely charged pairs at any given pH, thus they do not form strong enough complexes.

According to the present invention the weak polyelectrolyte can be either cationic or anionic. Preferably, the weak polyelectrolyte is a polysaccharide (glucosaminoglucan), the molecular weight of which is not larger than 100 000 Da. There are many weak polyelectrolytes of biological origin, such as alginate (Alg) or hyaluronic acid (HA). Non-biological weak polyanions are for example polyacrylic acid (PAA) or polycarboxylic acid (PCA). Non-biological weak polycations are for example polyethylene imine (PEI) or polyallyl amine hydrochloride (PAH).

According to the present invention, the used strong polyelectrolyte, the dissociation state of which is independent of pH, and is fully dissociated at all relevant pHs, can also be either a polyanion or a polycation. Strong polyanions are for example polystyrene sulfonate (PSS), or the natural polyelectrolytes chondroitin sulfate (KS) and heparan sulfate (HS). There are many examples of strong polycations, such as poly-diallyl dimethyl ammonium hydrochloride (PDDA) or poly-L-lysine (PLL). A strong cationic polyelectrolyte of biological origin is for example, a modification of chitosan obtained by grafting some cationic functional group such as trimethyl ammonium cation or pyridinium cation. Chitosan functionalization is relatively simple to perform, the esterification reaction does not require peculiar conditions and diverse functional groups can be added.

Appropriate polyelectrolyte pairs are, for example, chitosan and PSS, chitosan and low molecular weight (5000 Da) KS, PEI and KS, PDDA and PAA, PAA and alginate or PLL and HA pairs. Using KS-chitosan pair such side effects as encysting of the polyelectrolytes after subcutan administration can be avoided. Blood clothing prevention effect of heparin, another sulfated glucosamino glucan, analogous to chondroitin sulfate, can be exploited in a joint mechanism Possible favourable effect of the use of PSS can be the curing effect of PSS itself, since it is generally applied to decrease the potassium level in blood.

For the preparations we use preferably such linear polyelectrolytes that are biocompatible, biodegradable or bioactive/bioreactive.

In a preferable embodiment one of the layer forming polyelectrolytes is polystyrene sulfonate (PSS) and the other is chitosan.

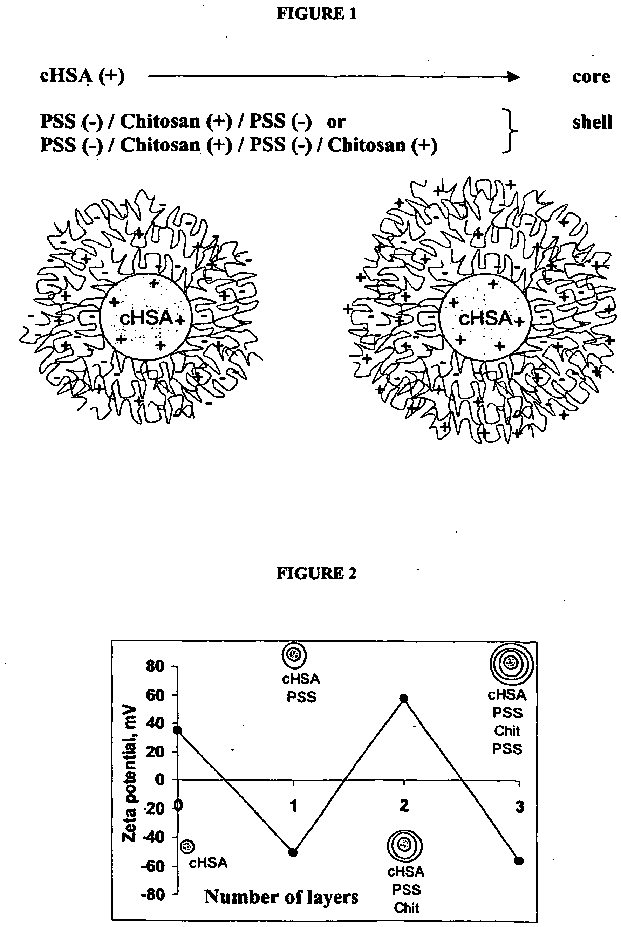

In a further preferable embodiment the nanocomposite of the invention consists of a cytokine containing human serum albumin (cHSA) core, that is stabilized by three polyelectrolyte layers, PSS, chitosan and PSS. Schematic representation of the structure of such formulation is shown in part (A) of Figure 1.

In a further aspect the present invention is directed to a process for the preparation of the above described nanocomposite in such a way that

i) to a solution of the bioactive protein or to a mixture containing at least one bioactive protein

a) an electrolyte solution with two- or three-valent anion is added during stirring at a pH, adjusted by using buffer solution to a value lower than the isoelectric point of the protein, or
b) an electrolyte solution with higher-valent cation is added while stirring at a pH, adjusted by using buffer solution to a value higher than the isoelectric point of the protein,

ii) the precipitated protein positively charged in case a) or negatively charged in case b) is separated via centrifugation,
iii) a solution of a first polyelectrolyte, oppositely charged compared to the precipitated protein, the pH of which adjusted with a buffer to the same value, is added to the protein sediment,
iv) the nanocomposite particles oppositely charged compared to the protein core, and coated with one polyelectrolyte layer are separated by centrifugation and optionally washed with buffer solution,
v) a solution of a second polyelectrolyte of opposite charge compared to the first polyelectrolyte, the pH of which adjusted to the same value using buffer, is added to the sediment while stirring,
vi) the nanocomposite particles having the same charge as the protein core and coated with two polyelectrolyte layers are separated by centrifugation and optionally washed with buffer, and vii) steps iii) - vi) are repeated until the desired number of layers is obtained, and finally
viii) the sediment of the core-shell nanocomposite having 3 to 5 number of layers as desired is freeze-dried.

Precipitation is conducted in a buffer medium to prevent protein denaturation. As buffers, both PBS (pH=7.4, consisting of 137 mM NaCl, 10 mM phosphate and 2.7 mM KCl) and acetate buffers can be applied.

Macromolecules can be coagulated with multivalent anions at a pH below, or with multivalent cations at a pH above the isoelectric point (iep). The two kinds of precipitation proceeds via different mechanisms. In acidic environment, the multivalent anions cause protein aggregation due to charge screening effect, through decreasing the thickness of electric double layer around the protein. At high pH, the multivalent cations hydrolyze and the precipitated polyoxo-hydroxo-complexes are flocculated by the oppositely charged protein molecules in the bridging flocculation mechanism, forming low density, voluminous flocs.

As a result of the acidic precipitation mechanism, primary protein particles of 10 to 100 nm diameter can be prepared with a high yield (>90 %), while cationic precipitation yields very low (10 to 30 %) protein load in the forming large (micrometer sized) protein-salt hydroxocomplex flocs.

The efficiency of the precipitation and the resulting particle size are sensitive to small shifts in the pH. The optimum value of pH depends on the isoelectric point of the protein and on the type of the electrolyte anion. 0.1 to 0.4 unit shift in pH is sufficient to either completely prevent precipitation at pH shift to the basic side, or to form non-controllable giant protein clots at pH shift to the acidic side.

For example, for precipitation of leukocyte interferon (LE-IFN) from aqueous media using sodium sulfate, the optimum value of pH is preferably in between 2.6 and 3.2, and the optimal molar ratio of $Na_2SO_4$ to LE-IFN is preferably in between 800 : 1 and 1200 : 1.

According to the present invention mechanic stirring is applied preferably in all of the separate phases of the preparation process. The speed of rotation during the stirring is 50 to 4000 rotations/min (rpm), preferably 80 to 100 rpm Use of ultrasonic agitation decreases the efficiency of precipitation.

In the end of the separate phases of the process particle separation is performed using centrifugation, preferably at 4000 to 7000 rpm.

Acetic acid, citric acid, lactic acid or acetate buffer at a concentration of 1 to 2 M are used to dissolve the polyelectrolytes. Instead of the buffers, HCl or NaOH solutions are also applicable to set the desirable pH values.

To form the adsorbed polyelectrolyte layers, the concentration of the polyelectrolyte solutions can be varied in a wide range, in general between $1 \times 10^{-6}$ and 1 % m/v. The value of the appropriate concentration in given specific cases is defined by various factors, such as the strength of the adsorption interaction between the surface to be coated and the polyelectrolyte, the molecular weight of the polyelectrolyte, the pH and the applied ionic strength. According to our observations the concentration of the polyelectrolyte necessary to form the first polyelectrolyte layer on the surface of the protein nanoparticles may be larger possibly by several orders of magnitude, than that of the subsequent polyelectrolyte layers.

As washing buffer solution acetate, citrate, tartaric acid, citric acid or ftalic acid buffers (pH 2 to 4) are used, at a concentration of 0.01 to 0.1 M.

We measured the zeta potential and the size of the particles after each preparation step by using dynamic light scattering (DLS) method. The sign of the zeta potential as shown in Figure 2, varied according to our expectations. It changed from positive to negative and back, corresponding to the charge sign of the outer polyelectrolyte layer. The size of the particles increased gradually with increasing number of polyelectrolyte layers. The size of the particles during the preparation steps changed as follows: 10 nm for primary precipitates of the cytokine containing protein, 50 to 80 nm for the nanoparticles coated with the first polyelectrolyte (PSS) layer, 100 to 300 nm for the second polyelectrolyte (chitosan), 300 to 600 nm for the third polyelectrolyte (PSS), and 600 to 900 nm for the fourth polyelectrolyte (chitosan) layer coated particles.

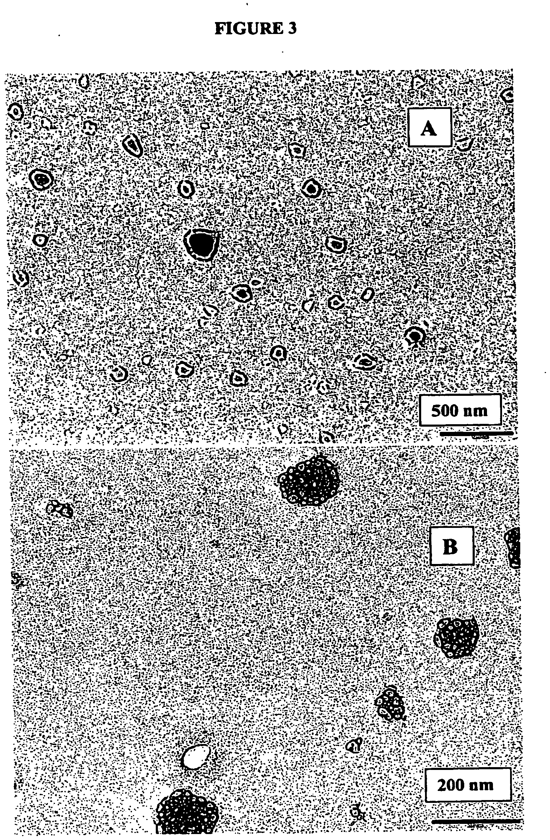

Transmission electron microscopic (TEM) pictures of the nanoparticles composed of cHSA core and PSS/chitosan/PSS three-layered shell, prepared according to the procedure of the present invention are presented in Figure 3. As the TEM pictures reveal, the size of the particles is smaller than 500 nm, and the size distribution is polydisperse. In all cases, the DLS sizes are larger than the TEM-derived sizes. The reason of this is, that DLS gives hydrodynamic diameters of the protein together with its hydration shell (the coating polyelectrolytes are strongly hydrated as well), while TEM gives the own size of the particles (at high vacuum in the TEM chamber).

In a further aspect the present invention directed to a sustained release drug formulation containing the pharmaceutically effective level of the nanocomposite of the invention, in combination with at least one pharmaceutically acceptable carrier and/or other excipient and optionally with other known pharmaceutically active drugs. The carrier and other excipient are "acceptable" in the sense, that they are compatible with the other ingredients of the formulation, and are not harmful to the recipient

A sustained release drug formulation of the present invention can be in any usual forms of administration which suits therapy. Administration can be oral, in the form of tablets, capsules, pills, powders or liquids, parenteral, in the various forms of injections (cutaneous, subcutaneous, intravenous or intramuscular), or via inhalation. The nanoparticles of the present invention are especially suitable for the preparation of intravenous or inhalation formulations because of their size. The drug formulations are prepared by using generally accepted methods, that are well known for a person skilled in the art.

For further details of the formulation and administration of drugs in general reference is made for example to the latest issue of Remington's Pharmaceutical Sciences (Mack Publishing Co., Easton, PA, USA).

For further details of the present invention, the following examples are given by way of illustration. All concentrations expressed in % mean % mass/volume (m/v) concentrations unless otherwise specified.

Example 1.a

Preparation of a nanocomposite consisting of Leukocyte IFN (LE-IFN) core and a three-layered polyelectrolyte shell

The starting material is freeze-dried interferon (Human Leukocyte Interferon (LE-IFN) of PBL Biomedical Laboratories, product number 11350-1), produced by blood leukocytes induced by Sendai virus, from which a solution is prepared at 4% with ion-free water filtered through 0.45 $\mu$m filter (ultrapure water). 10 mL of said LE-IFN solution is brought to pH 3.5 with hydrochloric acid and 3 mL of $Na_2SO_4$ solution (1.6 M) is added to the solution while stirring with a magnetic stirrer. Stirring is continued for 30 minutes, during which time more than 90 % of the protein precipitates. The formed protein nanoparticles are centrifuged at 5000 rpm for 15 minutes.

The supernatant of the sediment is removed and the protein nanoparticles of the sediment are dispersed in 10 mL of polystyrene sulfonate [PSS, M = 200000 Da (Aldrich)] solution of 0.2 % concentration the pH of which is set to 3.5 using HCl. The dispersion is stirred vigorously for 20 minutes (using magnetic stirrer) and then centrifuged at 5000 rpm for 15 minutes to separate the LE-EFN/PSS particles.

The supernatant is removed from the sediment of LE-IFN/PSS particles and the sediment is dispersed in chitosan (medium molecular weight, Aldrich) solution of 0.2 % concentration, the pH of which is set to 3.5 with HCl. After vigorous stirring for 20 minutes with a magnetic stirrer the LE-IFN/PSS/chitosan particles are centrifuged at 5000 rpm for 15 minutes.

The supernatant is removed from the sediment of LE-IFN/PSS/chitosan particles and the sediment is dispersed in 10 mL of polystyrene sulfonate (PSS, M= 200000 Da) solution of 0.2 % concentration the pH of which is set to 3.5 using HCl. After vigorous stirring for 20 minutes (using magnetic stirrer) the LE-IFN/PSS/chitosan/PSS particles are centrifuged at 5000 rpm for 15 minutes.

The supernatant of the sediment of LE-IFN/PSS/chitosan/PSS particles is removed and the sediment is dispersed in 1 mL HCl solution at pH 3.5. The final product is obtained form the dispersion by freeze-drying.

Example 1.b

**Preparation of a nanocomposite consisting of Leukocyte IFN (LE-IFN) core and a three-layered polyelectrolyte shell**

The starting material is freeze-dried interferon (Human Leukocyte Interferon (LE-IFN) of PBL Biomedical Laboratories, product number 11350-1), produced by blood leukocytes induced by Sendai virus, from which a solution is prepared at 4% with ion-free water filtered through 0.45 $\mu$m filter (ultrapure water). 10 mL of said LE-IFN solution is set to pH 3.5 with hydrochloric acid and 3 mL of $Na_2SO_4$ solution of 1.6 M concentration is added to the solution while stirring with a magnetic stirrer. Stirring is continued for 30 minutes, during which time more than 90 % of the protein precipitates. The formed protein nanoparticles are centrifuged at 5000 rpm for 15 minutes.

The supernatant of the sediment is removed and the protein nanoparticles of the sediment are dispersed in 10 mL of polystyrene sulfonate [PSS, M = 200000 Da (Aldrich)] solution of 0.2 % concentration the pH of which is set to 3.5 using HCl. The dispersion is stirred vigorously for 20 minutes (using magnetic stirrer) and then centrifuged at 5000 rpm for 15 minutes to separate the LE-IFN/PSS particles.

The supernatant is removed from the sediment of LE-IFN/PSS particles and the sediment is dispersed in chitosan (medium molecular weight, Aldrich) solution of $0.2 \times 01^{-4}$ % concentration - being 4 orders of magnitude smaller than in Example 1.a - the pH of which is set to 3.5 with HCl. After vigorous stirring for 20 minutes with a magnetic stirrer the LE-EFN/PSS/chitosan particles are centrifuged at 5000 rpm for 15 minutes.

The supernatant is removed from the sediment of LE-IFN/PSS/chitosan particles and the sediment is dispersed in 10 mL of polystyrene sulfonate (PSS, M= 2000000 Da) solution of $0.2 \times 01^{-4}$ % concentration - being 4 orders of magnitude smaller than in Example 1.a - the pH of which is set to 3.5 using HCl. After vigorous stirring for 20 minutes (using magnetic stirrer) the LE-IFN/PSS/chitosan/PSS particles are centrifuged at 5000 rpm for 15 minutes.

The supernatant of the sediment of LE-IFN/PSS/chitosan/PSS particles is removed and the sediment is dispersed in 1 mL HCl solution the pH of which is 3.5. The final product is obtained form the dispersion by freeze-drying.

The product prepared according to Example 1.b showed the same characteristics regarding particle size, zeta potential, release kinetics and biological activity of the encapsulated drug as the product prepared according to Example 1.a.

Example 2

Preparation of a nanocomposite consisting of Leukocyte IFN (LE-IFN) core and a four-layered polyelectrolyte shell

The procedure described in Example 1.a is repeated, with the exception that the centrifuged LE-IFN/PSS/chitosan/PSS nanoparticles are further dispersed in 10 mL of chitosan solution of 0.2 % concentration, the pH of which is set to 3.5 using HCl. The dispersion is stirred vigorously for 20 minutes (using magnetic stirrer) and the LE-IFN/PSS/chitosan/PSS/chitosan nanoparticles are cenrifuged at 5000 rpm for 15 minutes.

The supernatant of the sediment containing the LE-IFN/PSS/chitosan/PSS/chitosan particles is removed and the particles are dispersed in I mL of HCl solution of pH 3.5. The final product is obtained by freeze-drying.

Nanocomposites according to Examples 3 to 9 are prepared by using essentially the above described procedures and varying parameters of the synthesis as listed in Table 1.

Table 1

| Synthesis parameters of the LE-IFN nanocomposites coated by PSS and chitosan polyelectrolytes by the L-b-L method | | | | | | | | | |
|---|---|---|---|---|---|---|---|---|---|
| **Example** | $c_{prot.}$ % | **pH** | **aa/ph** | **disp** | **stirr** | **layer** | **prod.** | **release** | **act.** |
| 3 | 3.0 | 3 | aa | buff | US | 3 | 10% | n.m. | $>1.6 \times 10^5$ |

(continued)

| Synthesis parameters of the LE-IFN nanocomposites coated by PSS and chitosan polyelectrolytes by the L-b-L method | | | | | | | | | |
|---|---|---|---|---|---|---|---|---|---|
| Example | $c_{prot.}$ % | pH | aa/ph | disp | stirr | layer | prod. | release | act. |
| 4 | 3.0 | 3 | aa | buff | US | 3 | 11% | n.m. | >1.6x10⁵ |
| 5 | 4.0 | 3 | aa | PE | stirr | 3 | 45% | lin | >1.6x10⁵ |
| 6 | 4.0 | 2,6 | aa | PE | stirr | 3 | 40% | lin | 1.6x10⁵ |
| 7 | 5.0 | 3 | ph | buff | stirr | 4 | 42% | sat | 1.13 |
| 8 | 5.0 | 5 | ph | buff | stirr | 4 | 40% | sat | 1.13 |
| 9 | 5.0 | 3 | ph | PE | stirr | 4 | 48% | sat | 1.6 |

Headings of Table 1:

$c_{prot}$ % - concentration of LE-IFN solution before precipitation, % m/v pH - pH of the precipitation and polyelectrolyte layer building up procedure aa/ph - the acid used to set pH: acetic acid / phosphoric acid disp - the dispersion medium after centrifugation: buffer (buff), acetic acid (aa) or phosphoric acid (ph) for washing, or directly the polyelectrolyte solutions (PE) PSS or chitosan to build the next layer stirr - the procedure of homogenization, stirring with magnetic stirrer (stirr) or ultrasound (US) layer - number of polyelectrolyte layers: 3 - PSS/chitosan/PSS, 4 - PSS/chitosan/PSS/chitosan prod. - percentage of production: g composite/100 g original protein release - characterization of release kinetics: linear (lin) or saturation (sat) curves (see Example 13), n.m. means non measurable act. - antiviral activity of the LE-IFN formulation (NE/mg composite) (see Example 14, and data in Table 2)

Example 10

**Preparation of a nanocomposite consisting of interleukin (IL) core and a three-layered polyelectrolyte shell**

A mixture of recombinant human interleukine-2 (rHuIL2, CytoShop) and human serum albumin is prepared in a ratio of 1:50, respectively, both proteins dissolved in PBS solution, and the mixture is freeze-dried. The freeze-dried protein mixture (protein) is dissolved at 4 % concentration in ion-free water filtered through 0.45 µm filter (ultrapure water). 10 mL of IL2 solution is set to pH 2.5 using acetic acid, and 2.5 mL of $Na_3PO_4$ solution of 0.2 M concentration is added to the solution while stirring with magnetic stirrer. The stirring is continued for 20 minutes. During stirring more than 90 % of the protein precipitates. The so-formed protein nanoparticles are centrifuged at 5000 rpm for 15 minutes.

The supernatant of the sediment is removed and the protein nanoparticles of the sediment are dispersed in 10 mL of chondroitin sulfate [KS, (Sigma)] solution of 0.05 % concentration set to pH 2.5 using acetic acid. The dispersion is stirred vigorously for 20 minutes (using magnetic stirrer) and then centrifuged at 5000 rpm for 15 minutes to separate the IL2/KS particles.

The supernatant is removed from the sediment of IL2/KS particles and the sediment is dispersed in PEI (Fluka, M = 600000) solution of 0.01 % concentration, the pH of which is set to 2.5 with HCl. After vigorous stirring for 20 minutes with magnetic stirrer the IL2/KS/PEI particles are centrifuged at 5000 rpm for 15 minutes.

The supernatant is removed from the sediment of IL2/KS/PEI particles and the sediment is dispersed in 10 mL of KS solution of 0.05 % concentration the pH of which is set to 2.5 using HCl. After vigorously stirring for 20 minutes (using magnetic stirrer) the IL2/KS/PEI/KS particles are centrifuged at 5000 rpm for 15 minutes.

The supernatant of the sediment of IL2/KS/PEI/KS particles is removed and the sediment is dispersed in 1 mL of acetic acid solution the pH of which is 2.5. The final product is obtained form the dispersion by freeze-drying.

Example 11

**Preparation of a nanocomposite containing TNF**

A mixture of recombinant human TNF alpha (rHuTNF-a, CytoShop) and human serum albumin is prepared in 1:100 ratio, respectively, both proteins dissolved in PBS solution, and the mixture is freeze-dried. The freeze-dried protein mixture (protein) is dissolved at 4 % concentration in ion-free water filtered through 0.45 µm filter (ultrapure water). 10 mL of TNF solution is set to pH 3.4 using acetic acid and 2.5 mL of $Na_2CO_3$ solution (1.8 M) is added to the solution during stirring with magnetic stirrer. The stirring is continued for 20 minutes, during which more than 90 % of the protein precipitates. The so-formed protein nanoparticles are centrifuged at 5000 rpm for 15 minutes.

The supernatant of the sediment is removed and the protein nanoparticles of the sediment are dispersed in 10 mL of HS [Sigma, fast moving fraction] solution of 0.15 % concentration set to pH 3 using acetic acid. The dispersion is stirred vigorously for 20 minutes (using magnetic stirrer) and then centrifuged at 5000 rpm for 15 minutes to separate the TFN/HS particles.

The supernatant is removed from the sediment of TFN/HS particles and the sediment is dispersed in 10 mL of PAH (Aldrich, M = 15000) solution of 0.15 % concentration set to pH 3.4 with HCl. After vigorous stirring for 20 minutes with magnetic stirrer the TNF/HS/PAH particles are centrifuged at 5000 rpm for 15 minutes.

The supernatant is removed from the sediment of TNF/HS/PAH particles and the sediment is dispersed in 10 mL of HS solution of 0.15 % concentration set to pH 3.4 using HCl. After vigorous stirring for 20 minutes (using magnetic stirrer) the TNF/HS/PAH/HS particles are centrifuged at 5000 rpm for 15 minutes.

The supernatant of the sediment of TNF/HS/PAH/HS particles is removed and the sediment is dispersed in 1 mL of acetic acid solution the pH of which is 3.4. The final product is obtained form the dispersion by freeze-drying.

Example 12

**Preparation of a nanocomposite consisting of Leukocyte, IFN (LE-IFN) core and a three-layered polyelectrolyte shell**

A solution of 4% concentration is prepared from the freeze-dried active agent LE-IFN with ion-free water filtered through 0.45 μm filter (ultrapure water). 10 mL of the LE-IFN solution is set to pH 8 with NaOH solution and 3 mL of LaCl$_3$ (Aldrich) solution of 0.014 M concentration is added to the protein solution during stirring with magnetic stirrer. Stirring is continued for 30 minutes, during which time more than 90 % of the protein precipitates. The formed protein nanoparticles are centrifuged at rotation speed of 5000 rpm for 15 minutes.

The supernatant of the sediment is removed and the protein nanoparticles of the sediment are dispersed in 10 mL of PDDA [Aldrich, medium molecular weight] solution of 0.2 % concentration set to pH 8 using NaOH. The dispersion is stirred vigorously for 20 minutes (using magnetic stirrer) and then centrifuged at 5000 rpm for 15 minutes to separate the LE-IFN/PDDA particles.

The supernatant is removed from the sediment of LE-IFN/PDDA particles and the sediment is dispersed in alginate (sodium alginate, Aldrich, viscosity 200000 - 400000 cP) solution of 0.2 % concentration, set to pH 8 with NaOH. After vigorous stirring for 20 minutes with magnetic stirrer the LE-IFN/PDDA/Alg particles are centrifuged at 3000 rpm for 15 minutes.

The supernatant is removed from the sediment of LE-IFN/PDDA/Alg particles and the sediment is dispersed in 10 mL of PDDA solution of 0.2 % concentration set to pH 8 using NaOH. After vigorous stirring for 20 minutes (using magnetic stirrer) the LE-IFN/PDDA/Alg/PDDA particles are centrifuged at 5000 rpm for 15 minutes.

The supernatant of the sediment of LE-IFN/PDDA/Alg/PDDA particles is removed and the sediment is dispersed in 1 mL NaOH solution of pH 8. The final product is obtained from the dispersion by freeze-drying.

Example 13

**Investigation of the release kinetics of the nanocomposites prepared according to the present invention**

The release kinetics studies were conducted as follows.

The LE-IFN/PSS/chitosan/PSS or LE-IFN/PSS/chitosan/PSS/chitosan bionanocomposites were dispersed at known concentrations in PBS buffer pH 6.8 and kept in a thermostate box at 37 °C. The suspensions were centrifuged after 24 hours. After centrifugation, the supernatants were analyzed by UV-VIS spectrometry for LE-IFN concentrations. Characteristic protein absorption maximum at wavelength (λ) of 278 nm was used for analysis. The sediments were redispersed in fresh buffer solutions and the suspension put back to 37 °C into the thermostate box again for 24 hours. The release experiments were continued for 7 days.

According to the generally accepted drug release kinetics investigation protocols the medium is not exchanged, but only is filled up with fresh buffer solution after sampling. We had observed in our experiments, that following the accepted protocol route, the medium had saturated with the protein after 24 hours, and no further release could be observed during the experiment time. To get the protein fully released, we had to exchange the medium totally every day. The method used by us for investigation of release kinetics is in harmony with the recommendations of the European and American Associations of Pharmacologists as presented in the Basel Communication in February 2003 [D. J. Burgess, D. J.A. Crommelin, A. S. Hussain, M.-L. Chen, Assuring Quality and Performance of Sustained and Controlled Release Parenterals: EUFEPS Workshop Report, AAPS PharmSci 2004; 6 (1) Article 11 (http://www.aapspharmsci.org)].

According to the above report in vitro release kinetics of parenteral formulations should be investigated in flow-through experimental setup, but if tested under static conditions, frequent exchange of the medium is suggested.

The details of the analytical method we applied are as follows. UV-VIS spectra of the supernatants contain not only the peaks characteristic of the absorbing chromophores of the amino acids, but also the PSS absorption at $\lambda=261$ nm. The concentration of LE-IFN under the above condition have to be calculated by deconvolution of the envelop (LE-IFN + PSS) curve. Absorption of protein at 278 nm was calculated from the following equations:

$$Abs_{261} = x\, \varepsilon_{(LE\text{-}IFN)261} + y\, \varepsilon_{(PSS)261}$$

$$Abs_{278} = x\, \varepsilon_{(LE\text{-}IFN)278} + y\, \varepsilon_{(PSS)278}$$

In the above equations $Abs_{261}$ and $Abs_{278}$ are the extinction of the (LE-INF + PSS) envelop curve at the given wavelengths. x and y are the unknown concentrations of LE-IFN and PSS, respectively. $\varepsilon_{LE\text{-}IFN}$ and $\varepsilon_{PSS}$ are the extinction coefficients of LE-IFN and PSS at the given wavelengths (278 or 261 nm). Solving the equations for x gives the concentration of the released protein drug in the LE-IFN and PSS mixture. PSS dissolves to some extent during the experiments.

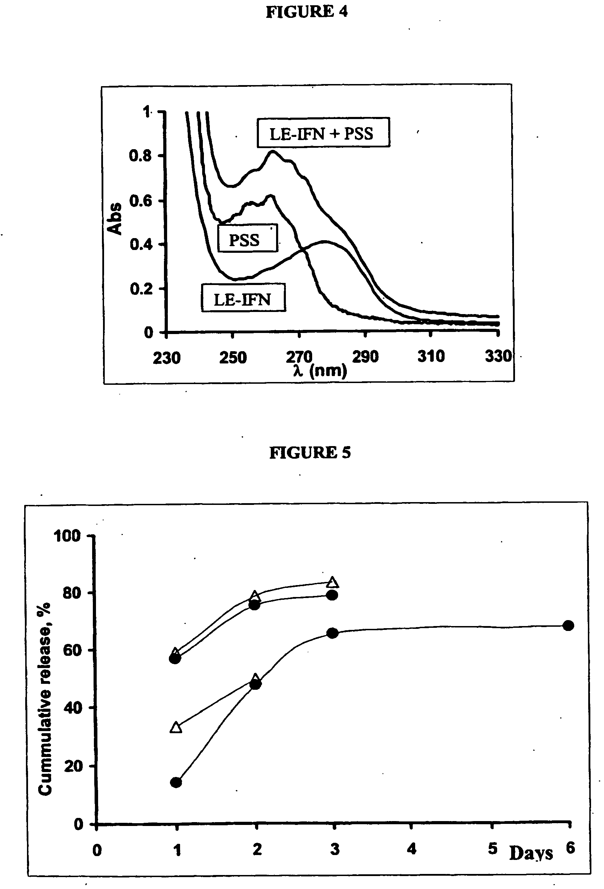

UV-VIS spectra of LE-IFN solution, PSS solution and of (LE-IFN + PSS) mixture are shown in Figure 4.

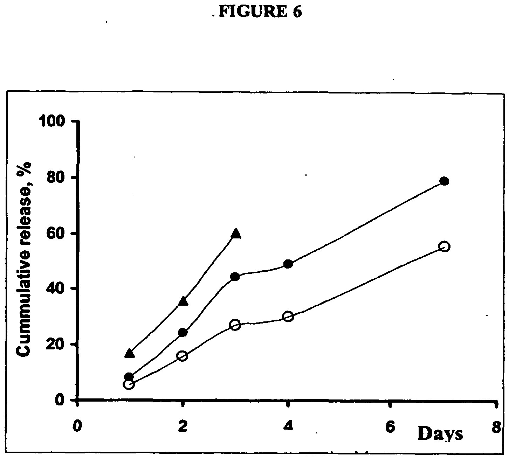

The release kinetic curves were calculated on the basis of the dissolved LE-IFN concentrations, normalized for the mass of the whole nanocomposite. Figures 5 and 6 represent the release kinetic curves of four and three polyelectrolyte layer coated nanocomposites, respectively. The samples were prepared according to the above general procedure, with small variations in the protocol as for example seen in Table 1. The release kinetics correlated with the number of polyelectrolyte layers, but it did not correlate with the small variations in the other experimental conditions. The change in the pH between 2.6 and 3, changes in the protein concentration between 3 and 4 %, dispersion of the particles between the layer building steps in either buffer or polyelectrolyte solution, using acetate or phosphate as buffer did not affect the release kinetics appreciably. Nanocomposites with the same number of coating polyelectrolyte layers had the same release character. According to the results, the formulations composed of protein core and the shell consisting of three polyelectrolyte layers are preferable for preparing sustained release formulations, since the initial (instant) release is less than 20 % and the daily increase in the release is nearly linear. On contrary, the four-layer composites show initial burst (instant release of the 20-60 % of the encapsulated drug) and the release reaches plateau with maximum release percentages at the 2nd and 3rd days.

Example 14

**Investigation of the biological activity of the nanocomposites of the present invention**

For determination of the biological activity in WISH tissue culture, Vesicular Stomatitis virus and NIH Ga23-902-530 Hu IFN-$\alpha$ standards were used. The results are summarized in Table 2.

Table 2

| Number | ID | Mass (mg) | Antiviral activity (NE/mg) | |
|---|---|---|---|---|
| I | LE-IFN freeze-dried | 109.9 | 1.92 x 10⁵ | |
| IA | | | 1.36 x 10⁵ | |
| II | Example 3 | 12.9 | >1.6 x 10⁵ | |
| IIA | | | >1.6 x 10⁵ | |
| III | Example 4 | 16.4 | >1.6 x 10⁵ | |
| IIIA | | | >1.6 x 10⁵ | **3-layered** |
| IV | Example 5 | 34.8 | >1.6 x 10⁵ | |
| IVA | | | 1.6 x 10⁵ | |
| V | Example 6 | 30.4 | 1.6 x 10⁵ | |
| VA | | | 1.6 x 10⁵ | |
| 1 | Example 7 | 102.2 | 1.13 x 10⁵ | |
| 1A | | | 8 x 10⁴ | |
| 2 | Example 8 | 94.9 | 1.13 x 10⁵ | **4-layered** |
| 2A | | | 8 x 10⁴ | |
| 3 | Example 9 | 131.5 | 1.6 x 10⁵ | |
| 3A | | | 5.64 x 10⁴ | |

Samples designated by "A" mean the part of the corresponding preparations passed through 0.45 $\mu$m filter membranes. The total LE-IFN contents of the composites were between 70 and 90 %, as obtained from material balance calculations during the preparation. Based on the above data we can state that the protein retained biological activity. According to the activity tests, the biological effect of the three-layered composites is superior compared with the four-layered composites. The activity of the protein formulated in three-layered nanocomposite is 85 % of the freeze-dried sample (from Table 2: $1,6x10^5/1,9x10^5=0,84$), while for the four-layered composites the same number is 60 % ($1,13x10^5/1,9x10^5=0,59$ or $8x10^5/1,36x10^5= 0,588$ for the filtered samples).

Example 15

**Pharmaceutical formulations**

a) A sustained release injection formulation was prepared from the nanocomposite of the invention with the following composition:

1 mg of the nanocomposite according to Example 1.a dispersed in
10 mL of PBS buffer solution.

b) A sustained release tablet formulation was prepared from the nanocomposite of the invention having the following composition:

50 mg of intermediate product (a 10 % dispersion of the nanocomposite according to Example 1.a in ion-free water, loaded onto ground cellulose surface using fluid bed procedure)
0.5 mg of magnesium stearate
72.5 mg of microcrystalline cellulose

Tablets having an average mass of 123 mg were pressed in a flat stamp of 8 mm diameter by applying 25 kN force.

13

# EP 2 170 302 B1

<u>Summary of the main advantages provided by the present invention</u>

According to the present invention, bioactive proteins, specifically cytokines, can be precipitated in a controlled way to form regular size, amorphous, primary nanoparticles, which are coated with polyelectrolyte multilayers using the L-b-L procedure. The present solution is suitable to control the particle size on demand, to prevent aggregation (colloid destabilization), to ensure controlled release kinetics (sustained release), to prevent denaturation of the encapsulated protein and to preserve biological activity.

The protein drugs are formulated generally as loads on carrier surfaces. Protein drugs, according to the present invention, can be formulated without carrier matrix as well, which has the benefit that in the primary particles the protein drug concentrations exceed that in the usual carrier-matrix formulations.

Further benefit of the present invention is that the proteins are not precipitated via desolvation mechanism, as in the case of the precipitations described in literature. Using the charge screening mechanism, primary particles of 10 nm diameter are formed very quickly, effectively and controllably. In the precipitation procedures known from literature, there are always used alcohols or other organic solvents as auxiliary coacervation agents, even in cases when the precipitation occurs due to pH-change or electrolyte addition. The organic solvent must be eliminated subsequently, which involves the danger of protein denaturation and requires additional time and energy input.

Further benefit of the present invention is that a 3-layered polyelectrolyte coating is sufficient to stabilize the protein nanoparticles and to ensure sustained protein release through this multilayer structure. As it is generally observed [F. Caruso, D. Trau, H. Mohwald, R. Renneberg, Enzyme Encapsulation in Layer-by-Layer Engineered Polymer Multilayer Capsules, Langmuir, 16, 1485-1488 (2000)], the usual polyelectrolyte coatings (e.g. 4 to 12 layer PSS-PAH structures) retain proteins of a size larger than 5 nm in diameter.

**Claims**

1. A sustained release nanocomposite, which comprises

   a) at least one cytokine drug precipitated ionically reversibly and forming a charged primary nanoparticle core and
   b) around the said core, a 3 to 5 layered polyelectrolyte shell, formed by alternate adsorption of oppositely charged polyelectrolytes the dissociation of one of which is pH-dependent, and the other is fully dissociated independently of pH.

2. The nanocomposite according to claim 1 containing interferon, interleukin, macrophage inflammation protein, tumor necrosis factor or the mixture of the above drugs as cytokine.

3. The nanocomposite according to claim 1 or claim 2, in which the core further contains a carrier protein.

4. The nanocomposite according to any of claims 1 to 3, which contains natural human interferon as cytokine and human serum albumin as carrier protein.

5. The nanocomposite according to any of claims 1 to 4, in which the protein core is precipitated using an electrolyte having a two- or three-valent anion at a pH below the isoelectric point of the protein or having a multivalent cation at a pH above the isoelectric point of the protein.

6. The nanocomposite according to claim 5, in which the core-forming protein is precipitated using sodium sulfate solution at pH below the isoelectric point of the protein.

7. The nanocomposite according to any of claims 1 to 6, in which the polyelectrolyte shell is formed by negatively charged polystyrene sulfonate and positively charged chitosan.

8. The nanocomposite according to claim 7, in which the positively charged core is surrounded by negatively charged polystyrene sulfonate / positively charged chitosan / negatively charged polystyrene sulfonate triple layer.

9. A sustained release pharmaceutical composition, which contains an effective amount of a nanocomposite according to any of claims t to 8 together with at least one pharmaceutically acceptable inert carrier and/or other excipient, and optionally one or more other, biologically active ingredients.

10. The use of a a nanocomposite according to any of claims 1 to 8, in the preparation of a sustained release pharmaceutical composition.

11. A process for the preparation of a nanocomposite according to any of claims 1 to 8, **characterized in that**

i) to a buffered aqueous solution of a cytokine active agent or a mixture thereof with a carrier protein

a) an electrolyte containing two- or three-valent anion at a pH below the isoelectric point of the protein(s) or
b) an electrolyte containing multivalent, non-hydrolyzing cation at a pH above the isoelectric point of the protein(s)

is added while stirring;
ii) the positively charged particles obtained in case a) or negatively charged particles obtained in case b) are isolated by centrifugation;
iii) a solution of an oppositely charged first polyelectrolyte the pH of which is buffered to the pH of the precipitated nanoparticles is added to the sediment obtained in the previous step while stirring;
iv) the nanocomposite particles coated with the first polyelectrolyte charged oppositely relative to the precipitated protein core, are separated by centrifugation and optionally washed with buffer;
v) a solution of a second polyelectrolyte, oppositely charged relative to the first polyelectrolyte layer, buffered to the same pH is added to the sediment obtained in the previous step while stirring;
vi) the nanocomposite particles coated with two polyelectrolyte layers, charged of the same sign as the core, are separated by centrifugation and optionally washed with buffer;
vii) steps iii) to vi) are repeated until the desired number of layers is formed by the first and the second polyelectrolytes one of which is dissociated dependently on the pH of the medium while the other is fully dissociated independently of pH;
and, finally,
viii) the sediment of the core-shell nanocomposite having 3 to 5 layers as desired is freeze-dried.


**Patentansprüche**

1. Nanokomposit mit verzögerter Freisetzung, welches

a) mindestens einen Cytokin-Wirkstoff, welcher ionisch reversibel ausgefällt und als ein geladener, primärer Nanopartikel-Kern geformt ist, und
b) um den erwähnten Kern eine 3 bis 5-schichtige Polyelektrolyt-Beschichtung, welche durch wechselnde Adsorption von gegensätzlich geladenen Polyelektrolyten geformt ist, wobei eine davon eine pH-abhängige und die andere eine vollkommen pH-unabhängige Dissoziation zeigt, enthält.

2. Nanokomposit nach Anspruch 1, welches als Cytokin Interferon, Interleukin, Makrophagenentzündungsprotein, Tumornekrosefaktor oder eine Mischung dieser enthält.

3. Nanokomposit nach Anspruch 1 oder Anspruch 2, wobei der Kern auch ein Trägerprotein enthält.

4. Nanokomposit nach einem der Ansprüche 1 bis 3, welches natürliches humanes Interferon als Cytokin und humanes Serumalbumin als Trägerprotein enthält.

5. Nanokomposit nach einem der Ansprüche 1 bis 4, wobei der Proteinkern durch Verwendung eines Elektrolytes, das bei einem pH-Wert unter dem isoelektrischen Punkt des Proteins ein zwei- oder dreiwertiges Anion oder bei einem pH-Wert über dem isoelektrischen Punkt des Proteins ein mehrwertiges Kation enthält, ausgefällt ist.

6. Nanokomposit nach Anspruch 5, wobei der Proteinkern durch Verwendung einer Natriumsulfat-Lösung bei einem pH-Wert unter dem isoelektrischen Punkt des Proteins ausgefällt ist.

7. Nanokomposit nach einem der Ansprüche 1 bis 6, wobei die Polyelektrolyt-Beschichtung mit negativ geladenem Polystyrensulfonat und positiv geladenem Chitosan geformt ist.

8. Nanokomposit nach Anspruch 7, wobei der positiv geladene Kern mit einer dreifachigen Beschichtung aus negativ geladenem Polystyrensulfonat/positiv geladenem Chitosan/negativ geladenem Polystyrensulfonat bezogen ist.

9. Pharmazeutische Zubereitung mit verzögerter Freisetzung, welche eine wirksame Menge an einem Nanokomposit nach einem der Ansprüche 1 bis 8 zusammen mit mindestens einem pharmazeutisch anwendbaren inerten Trägerstoff und/oder anderen Hilfsstoffen und gegebenenfalls einem oder mehreren weiteren pharmazeutischen Wirkstoffen enthält.

10. Verwendung eines Nanokomposits nach einem der Ansprüche 1 bis 8 zur Herstellung einer pharmazeutischen Zubereitung mit verzögerter Freisetzung.

11. Verfahren zur Herstellung eines Nanokomposits nach einem der Ansprüche 1 bis 8, **dadurch gekennzeichnet, dass** man

i) einer gepufferten wässrigen Lösung von Cytokin-Wirkstoff oder einer Mischung von diesem mit einem Trägerprotein unter Rühren

a) bei einem pH-Wert unter dem isoelektrischen Punkt des Proteins oder der Proteine einen, ein zwei- oder drei-wertiges Anion enthaltenden Elektrolyten oder
b) bei einem pH-Wert über dem isoelektrischen Punkt des Proteins oder der Proteine einen, ein mehrwertiges, nicht-hydrolisierendes Kation enthaltenden Elektrolyten
zusetzt;

ii) die nach Punkt (a) gewonnenen positiv geladenen Partikel oder die nach Punkt (b) gewonnenen negativ geladenen Partikel durch Zentrifugieren isoliert;
iii) dem im vorhergehenden Schritt gewonnenen Sediment unter Rühren eine, auf einen gleichen pH-Wert gepufferte Lösung eines ersten Polyelektrolyten mit entgegengesetzter Ladung wie die der ausgefällten Nanopartikel zusetzt;
iv) die mit dem eine entgegengesetze Ladung wie das ausgefällte Protein aufweisenden ersten Polyelektrolyten bezogenen Nanokomposit-Partikel durch Zentrifugieren isoliert und gegebenenfalls mit Puffer wäscht;
v) dem im vorhergehenden Schritt gewonnenen Sediment unter Rühren eine, auf einen gleichen pH-Wert gepufferte Lösung eines zweiten Polyelektrolyten mit entgegengesetzter Ladung wie die des ersten Polyelektrolyten zusetzt;
vi) die mit zwei Polyelektrolyt-Schichten bezogenen Nanokomposit-Partikel mit der gleichen Ladung wie der Kern durch Zentrifugieren isoliert und gegebenenfalls mit Puffer wäscht;
vii) die Schritte iii) bis vi) wiederholt, bis die gewünschte Anzahl Schichten von ersten und zweiten Polyelektrolyten erreicht ist, wobei eine davon eine pH-abhängige und die andere eine vollkommen pH-unabhängige Dissoziation zeigt; und schließlich
viii) das die gewünschten 3 bis 5 Schichten enthaltende Nanokomposit-Sediment mit Kern-Hülle-Struktur, gefriertrocknet.

**Revendications**

1. Nanocomposite à libération prolongée, comprenant

a) au moins un médicament cytokinique précipité ioniquement réversiblement et formant un noyau nanoparticule primaire chargé et
b) autour de ce noyau, une enveloppe polyélectrolytique à 3-5 couches formée par l'adsorption alternée de polyélectrolytes de charges opposés, la dissociation de l'un étant dépendent du pH et l'autre étant complètement dissocié indépendamment du pH.

2. Nanocomposite selon la revendication 1 contenant d'interféron, d'interleukine, de la protéine inflammatoire macrophagique, du facteur de nécrose tumorale ou le mélange de ces médicaments comme cytokine.

3. Nanocomposite selon la revendication 1 ou 2, dans lequel le noyau contient aussi une protéine porteuse.

4. Nanocomposite selon l'une quelconque des revendications 1 à 3 contenant d'interféron humain naturel comme cytokine et du sérum albumine humaine comme protéine porteuse.

5. Nanocomposite selon l'une quelconque des revendications 1 à 4, dans lequel le noyau protéique est précipité en utilisant un électrolyte comprenant un anion di- ou trivalent au pH au-dessous du point isoélectrique de la protéine, ou comprenant un cation multivalent au pH au-dessus du point isoélectrique de la protéine.

6. Nanocomposite selon la revendication 5 dans lequel la protéine formant le noyau est précipitée en utilisant de la solution de sulfate de sodium à un pH au-dessous du point isoélectrique de la protéine.

7. Nanocomposite selon l'une quelconque des revendications 1 à 6, dans lequel l'enveloppe polyélectrolytique est formée par du sulfonate de polystyrène chargé négativement et du chitosan chargé positivement.

8. Nanocomposite selon la revendication 7 dans lequel le noyau chargé positivement est entouré par une triple couche du sulfonate de polystyrène chargé négativement / du chitosan chargé positivement / du sulfonate de polystyrène chargé négativement.

9. Composition pharmaceutique à libération prolongée, contenant une quantité effective du nanocomposite selon l'une quelconque des revendications 1 à 8 en combinaison avec au moins une véhicule et/ou un autre excipient inerte pharmaceutiquement acceptable et optionnellement un ou plusieurs d'autres ingrédients biologiquement actifs.

10. Utilisation du nanocomposite selon l'une quelconque des revendications 1 à 8 pour la préparation d'une composition pharmaceutique à libération prolongée.

11. Procédé pour la préparation du nanocomposite selon l'une quelconque des revendications 1 à 8, **caractérisé en ce que**

i) à une solution aqueuse tamponnée d'un principe actif cytokinique ou de son mélange avec une protéine porteuse
est ajouté, tout en agitant

a) un électrolyte comprenant un anion di- ou trivalent au pH au-dessous du point isoélectrique de la protéine / des protéines, ou
b) un électrolyte comprenant un cation multivalent non-hydrolysant au pH au-dessus du point isoélectrique de la protéine / des protéines,

ii) les particules chargées positivement obtenues dans le cas a) ou les particules chargées négativement obtenues dans le cas b) sont isolées par centrifugation;
iii) une solution d'un premier polyélectrolyte de charge opposé dont le pH est tamponné au pH des nanoparticules précipités est ajoutée, tout en agitant au sédiment obtenu dans l'étape précédente;
iv) les particules nanocomposite enveloppées du premier polyélectrolyte de charge opposé par rapport du noyau protéique précipité sont isolées par centrifugation et optionnellement lavées par du tampon;
v) une solution d'un second polyélectrolyte de charge opposé par rapport du premier polyélectrolyte, tamponnée au même pH, est ajouté, tout en agitant au sédiment obtenu dans l'étape précédente;
vi) les particules nanocomposite enveloppées de deux couches de polyélectrolyte, ayant le même type de charge que le noyau sont isolées par centrifugation et optionnellement lavées par du tampon;
vii) les étapes iii) à vi) sont répétées jusqu' à la formation du numéro désiré des couches par les premier et second polyélectrolytes dont l'un étant dissocié en fonction du pH du milieu et l'autre étant complètement dissocié indépendamment du pH;
et finalement
viii) le sédiment du nanocomposite noyau-enveloppe ayant 3 à 5 couches est lyophilisé comme désiré.

**FIGURE 1**

cHSA (+) ————————————————➤ core

PSS (-) / Chitosan (+) / PSS (-)  or
PSS (-) / Chitosan (+) / PSS (-) / Chitosan (+) } shell

**FIGURE 2**

**FIGURE 3**

500 nm

200 nm

FIGURE 4

FIGURE 5

.FIGURE 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0052510 A
- SE 462780
- US 5000886 A
- US 5019400 A
- WO 9013780 A
- US 4166800 A
- US 4568559 A
- US 5407609 A
- US 4384975 A
- WO 9607399 A
- US 4652441 A
- US 5654010 A
- WO 9640074 A
- US 5589167 A
- US 6861064 B
- US 5904936 A
- WO 2006106520 A
- US 2005239167 A

### Non-patent literature cited in the description

- **N. Jovanovic ; A. Bouchard ; G. W. Hofland ; G.-J. Witkamp ; D. J. A. Crommelin ; W. Jiskoot.** *Stabilization of Proteins in Dry Powder Formulations Using Supercritical Fluid Technology, Review, Pharmaceutical Research,* 2004, vol. 21, 1955-1969
- **P. Chattopadhyay ; R. B. Gupta.** Protein Nanoparticles Formation by Supercritical Antisolvent with Enhanced Mass Transfer. *AIChE Journal,* 2002, vol. 48, 235-244
- Nanotechnologies for life sciences. Wiley-VCH, Verlag GmbH & Co, 2006, vol. 2
- **U. Gietza ; T. Arvinte ; M. Haner ; U. Aebi ; H. P. Merkle.** Formulation of sustained release aqueous Zn-hirudin suspensions. *European Journal of Pharmaceutical Sciences,* 2000, vol. 11, 33-41
- **N. G. Balabushevitch ; G. B. Sukhorukov ; N. A. Moroz ; D. V. Volodkin ; N. I. Larionova ; E. Donath ; H. Mohwald.** Encapsulation of Proteins by Layer-by-Layer Adsorption of Polyelectrolytes onto Protein Aggregates: Factors Regulating the Protein Release. *Biotechnology and Bioengeneering,* 2001, vol. 76, 207-213
- **F. Caruso ; D. Trau ; H. Mohwald ; R. Renneberg.** Enzyme Encapsulation in Layer-by-Layer Engineered Polymer Multilayer Capsules. *Langmuir,* 2000, vol. 16, 1485-1488
- **T. Mauser ; C. De jugnat ; H. Möhwald ; G. B. Sukhorukov.** Microcapsules Made of Weak Polyelectrolytes: Templating and Stimuli-Responsive Properties. *Langmuir,* vol. 22, 5888-5893
- **D. V. Volodkin ; N. I. Larionova ; G. B. Sukhorukov.** Protein Encapsulation via Porous CaCO3 Microparticles Templating. *Biomacromolecules,* 2004, vol. 5, 1962-1972
- **H. Ai ; J. J. Pink ; X. Shuai ; D. A. Boothman ; J. Gao.** Interactions between self assembled polyelectrolyte shells and tumor cells. Wiley Periodicals, Inc, 2005
- **L. Jorgensen ; E. H. Moeller ; M. van de Weert ; H. M. Nielsen ; S. Frokjaer.** Preparing and evaluating delivery systems for proteins. *European Journal of Pharmaceutical Sciences*
- **Balabushevitch N.G. et al.** Encapsulation of proteins by layer-by-layer adsorption of polyelectrolytes onto protein aggregates: factors regulating the protein release. *Biotechnology and Bioengineering,* 2001, vol. 76, 207-213
- Remington's Pharmaceutical Sciences. Mack Publishing Co,
- **D. J. Burgess ; D. J. A. Crommelin ; A. S. Hussain ; M.-L. Chen.** Assuring Quality and Performance of Sustained and Controlled Release Parenterals: EUFEPS Workshop Report. *AAPS PharmSci,* 2004, vol. 6 (1, www.aapspharmsci.org